# EUROPEAN PATENT APPLICATION

(11) **EP 4 495 938 A1**
(43) Date of publication of application: **22.01.2025**
(21) Application number: 23186420.8
(22) Date of filing: 19.07.2023
(51) Int. Cl.: G16H 10/60, G06F 21/62

(54) **DISTRIBUTED CLINICAL DATA MANAGEMENT SOLUTION WITH PATIENT ID PROTECTION**

(71) Applicant: Koninklijke Philips N.V., 5656 AG Eindhoven (NL)
(72) Inventor: ZHANG, Jinsheng, Eindhoven (NL); CHEUNG, Yee Him, Eindhoven (NL)
(74) Representative: Philips Intellectual Property & Standards

(57) **Abstract**

Each medical site visited by a patient stores the patient's clinical data in a local database, indexed by a site-specific pseudo-ID (SPID) assigned to the patient, such as a patient number or other identifier. A Patient Data Directory System (PDDS) is provided to facilitate access to the clinical data of the patient at each medical site. As each medical site stores the patient's clinical data, a set of metadata is provided to the PDDS that identifies where the clinical data is stored at the medical site, as well as the SPID used at the particular site. This metadata is entered into the PDDS, and can be used to retrieve the clinical data stored at each medical site. Access to the PDDS is controlled by the patient, thereby enabling the patient to provide some or all of the metadata required to access the patient's clinical data to select clinicians. A variety of cryptographic protocols are used to prevent unauthorized access to the patient's data.

## Description

### FIELD OF THE INVENTION

This invention relates to the field of medical data management, and in particular to a system that enables management of a patient's clinical data that is distributed among a plurality of medical facilities while protecting the identity of the patient.

### BACKGROUND OF THE INVENTION

One of the biggest problems faced by the healthcare industry is the efficient management and prompt access to a patient's clinical data residing on multiple heterogeneous medical systems. With clinical data scattered over different medical sites, the patient typically does not have a complete view of his/her medical history. Additionally, due to lack of full control over these medical records, the patient is unaware of how the clinical data in the records are utilized. In like manner, it is difficult for clinicians to make correct and informed decisions without a full picture of the patient's medical profile.

Conventionally, a patient's record at each medical site may be uploaded to a 'cloud' server, and accessed as required by clinicians having access to the server. However, such a consolidation of records from a variety of medical sites incurs substantial overhead at the sites, typically requiring a transformation of the data at each site to conform to the requirements of the cloud storage facility with regard to the structure and format of the information being stored, and requires a patient identifier that is common to each of the medical sites. Typically, the common patient identifier is an identifier that is unique to the patient, such as the patient's National ID or Social Security Number. Additionally, to avoid the risk of a 'single point of failure', multiple redundant storage facilities must be provided.

Of particular concern, a data breach at a consolidated data storage facility may result in the unauthorized release of the patient's identity and the patient's entire medical history.

### SUMMARY OF THE INVENTION

Since clinical data contain highly sensitive information, a solution is required that enables linking the medical records of the patient at the multiple medical sites, while at the same time protecting the patient's privacy to the greatest extent possible.

To better address one or more of these concerns, it would be advantageous to provide a solution that enables each medical site to locally store the patient's clinical data using a locally defined identifier of the patient, preferably a site-specific pseudo-ID. Although each site must maintain a mapping of the local pseudo-ID to the actual identity of the patient, this mapping may be stored securely in a database that is independent of the database that is used to store the clinical data. Additionally, because each medical site assigns a local pseudo-ID to the patient, a data breach of the pseudo-ID to actual patient identity mapping at one site will not affect the security of the patient's identity, or the security of the clinical data, at any other site.

In an embodiment of this invention, each medical site stores the patient's clinical data in a local database, indexed by the patient's site-specific pseudo-ID.

To facilitate a linking of the patient's clinical data among each of the medical sites that contain the patient's clinical data, a Patient Data Directory System (PDDS) is provided. As each medical site stores the patient's clinical data, a set of metadata is provided that identifies where the clinical data is stored at the medical site, as well as other information, such as the patient's pseudo-ID at the particular medical site, that facilitates retrieval of this clinical data, and other data that serves to indicate what information is contained in the clinical data, such as a medical code that identifies the anatomical region related to the clinical data. This metadata is entered into the PDDS, indexed by a patient-selected identifier, such as a PIN.

In an embodiment of this invention, the metadata includes the URL (Universal Record Locator) of the particular site, the site-specific pseudo-ID of the patient, and other data as required to facilitate retrieval of the clinical data, such as cryptographic keys.

Also in an embodiment of this invention, the patient controls the access to and retrieval of the metadata, thereby controlling what information is released to any particular clinician. In this embodiment, the clinician that generates and stores the clinical data at a site provides the metadata to the patient, and the patient stores the metadata in the PDDS. When a clinician has a need for the patient's clinical data at other medical sites, the clinician contacts the patient, the patient selectively provides the metadata related to the clinician's request, and the clinician retrieves the clinical data at the other sites using this metadata.

In an embodiment, the identity of each clinician is authenticated, and an Authentication Token is issued to the clinician upon successful authentication. This Authentication Token is used to identify the clinician to the clinical sites, and to determine whether the authenticated clinician is authorized to gain access to the clinical data stored at each site that is identified by the metadata provided by the patient. When the patient first goes to a clinician, the clinician also uses the Authentication Token to register the patient and initiate the patient's access to the PDDS.

In an embodiment, the patient data directory system (PDDS) comprises a plurality of records associated with a patient, and an access control system that controls access to the plurality of records, wherein access to the plurality of records is controlled by the patient. The patient can approve or deny access to the clinical data of the patient by each of a plurality of data requestors. The plurality of records comprises metadata that is associated with the clinical data of the patient that is stored at a plurality of clinical sites. Each clinical site identifies the patient using a site-specific pseudo-ID (SPID) of the patient, and the metadata of each record identifies a location of the patient's clinical data at a corresponding clinical site, and the SPID of the patient at the corresponding clinical site. The access control system comprises an identity verification process to verify that a user of the system is the patient that controls access to the metadata records. If the user is verified as being the patient, and data access is approved for the data requestor, the metadata is communicated to the data requestor to enable the data requestor to access to the clinical data of the patient at the corresponding clinical site based on the SPID of the patient at the corresponding clinical site. As detailed further below, the PDDS may communicate the metadata to the patient, and the patient forwards the metadata to the data requestor.

In embodiments, a patient device comprises a communication system for communicating with a data requestor and the PDDS. The patient device includes a storage system, a cryptographic system, and a user interface. In use, the patient device receives a record-update request from the data provider and presents the request to the patient for approval or denial. The patient device uses the cryptographic system to gain access to the PDDS, and uses the communication system to communicate the metadata to the PDDS.

The patient device subsequently receives a record-access request from a data requestor and presents the request to the patient for approval or denial. If the request is approved, the patient device uses the cryptographic system to gain access to the metadata records of the patient at the PDDS, and, in response to the record-access request, receives the metadata records of the patient from the PDDS. The patient device selectively communicates some or all of the metadata to the data requestor, to facilitate access to the clinical data of the patient stored at the plurality of clinical sites by the data requestor.

In embodiments, a data provider device comprises a communication system for communicating with a patient, the PDDS, and a clinical site server associated with the data provider. The data provider device stores the patient's clinical data in a clinical site server and receives corresponding metadata from the server in response. The data provider device communicates patient verification information to the PDDS if the patient is new to the PDDS, and communicates a record-update request (metadata storage request) including the metadata to the patient for subsequent submission to the PDDS.

In embodiments, a data requestor device comprises a communication system for communicating with a patient and a plurality of clinical sites. The data requestor device communicates a data access request to the patient, and receives the metadata associated with the clinical data from the PDDS via the patient. The data requestor device subsequently accesses the clinical data of the patient stored at the plurality of clinical sites based on the received metadata.

In embodiments, a data access tracking system comprises a communication system for communicating with a plurality of patients, data providers, data requestors, clinical sites, and the PDDS. The data access tracking system includes a storage system, a cryptographic system, and a user interface. The data access tracking system stores access records associated with interaction messages among the plurality of patients, data providers, data requestors, clinical sites, and the PDDS. The interaction messages comprise, for example, a registration request, a record access request, a record update request, a clinical data access request, a request approval or denial, etc. Each access record includes information associated with each interaction message and a timestamp, and the user interface of the data access tracking system facilitates review of the access records.

Additionally, embodiments of this invention include a method of managing access to clinical records of a patient, the method comprising storing a plurality of records that facilitate access to the clinical records of the patient, wherein the plurality of records comprises metadata that is associated with the clinical records of the patient at a plurality of clinical sites, wherein the metadata of each record comprises an identification of a clinical site of the plurality of clinical sites and a pseudo-ID associated with the patient at the clinical site, and wherein the pseudo-ID is necessary to access the clinical data at the clinical site. The method further comprises receiving a record access request from a user, verifying that the user is the patient, and if the user is verified to be the patient, providing the metadata to at least one of: the patient, or a data requestor that is authorized by the patient to access the clinical data at the clinical site identified in the metadata.

Embodiments of this feature include a computer program that, when executed by a processing system, causes the processing system to perform the above method.

A variety of security measures may be applied to further improve the security of particular aspects of this invention, as detailed further below.

### BRIEF DESCRIPTION OF THE DRAWINGS

The invention is explained in further detail, and by way of example, with reference to the accompanying drawings wherein:
FIG. 1 illustrates an overview of the architecture of an example embodiment of this invention.
FIG. 2 illustrates an example of a clinician authentication system.
FIG. 3 illustrates an example message exchange for initiating a patient's access to the PDDS.
FIG. 4 illustrates an example flow diagram for reading or writing metadata on the PDDS.
FIG. 5 illustrates an example flow diagram for providing the patient's clinical data to the clinician, based on the patient's metadata from the PDDS.
FIG. 6 illustrates an example flow diagram for enabling the re-encryption of records in the PDDS.
FIG. 7 illustrates a flow diagram of an example method for providing access to a patient's metadata records that are stored at the PDDS.

Throughout the drawings, the same reference numerals indicate similar or corresponding features or functions. The drawings are included for illustrative purposes and are not intended to limit the scope of the invention.

### DETAILED DESCRIPTION

In the following description, for purposes of explanation rather than limitation, specific details are set forth such as the particular architecture, interfaces, techniques, etc., in order to provide a thorough understanding of the concepts of the invention. However, it will be apparent to those skilled in the art that the present invention may be practiced in other embodiments, which depart from these specific details. In like manner, the text of this description is directed to the example embodiments as illustrated in the Figures, and is not intended to limit the claimed invention beyond the limits expressly included in the claims. For purposes of simplicity and clarity, detailed descriptions of well-known devices, circuits, and methods are omitted so as not to obscure the description of the present invention with unnecessary detail.

For ease of understanding, the invention presented herein is presented in the context of a patient data directory that is controlled by the patient to facilitate access by clinicians to clinical data related to the patient. One of skill in the art will recognize, however, that the principles of this invention can be applied to other environments and applications.

Accordingly, the terms 'patient', 'clinician', 'clinical data', etc. are used in this context, but are herein defined to be generic terms for 'first participant', 'second participant', 'data associated with the first participant', etc. In like manner, the term 'patient' is defined herein to include a party that is authorized by the patient/'first participant' to access the data directory on behalf of the patient/'first participant', and the term 'clinician' is defined herein to include a party that is authorized to access the clinical data associated with the patient/'first participant'.

One of skill in the art will also recognize that the actions of the patient, the clinician, the site, etc. described herein will generally be performed using a device configured to perform the actions of the patient, a device configured to perform the actions of the clinician, a device/server configured to perform the actions of the site, etc. Thus, the terms 'patient' and 'patient device'; 'clinician' and 'clinician device; 'site' and 'site server', etc. may be used interchangeably herein. Similarly, a clinician may be a 'data provider' who provides the metadata that is to be stored using a 'data provider device', or a 'data requestor' who requests and receives the stored metadata using a 'data requestor device'. The data provider device and data requestor device may be embodied as a single component.

In like manner, one of skill in the art will recognize that the particular cryptographic methods presented in the example embodiments may be replaced by other cryptographic methods. For example, where feasible, a disclosed symmetric encoding method may be replaced by an asymmetric encoding method to encrypt the material to be protected from disclosure, and vice versa.

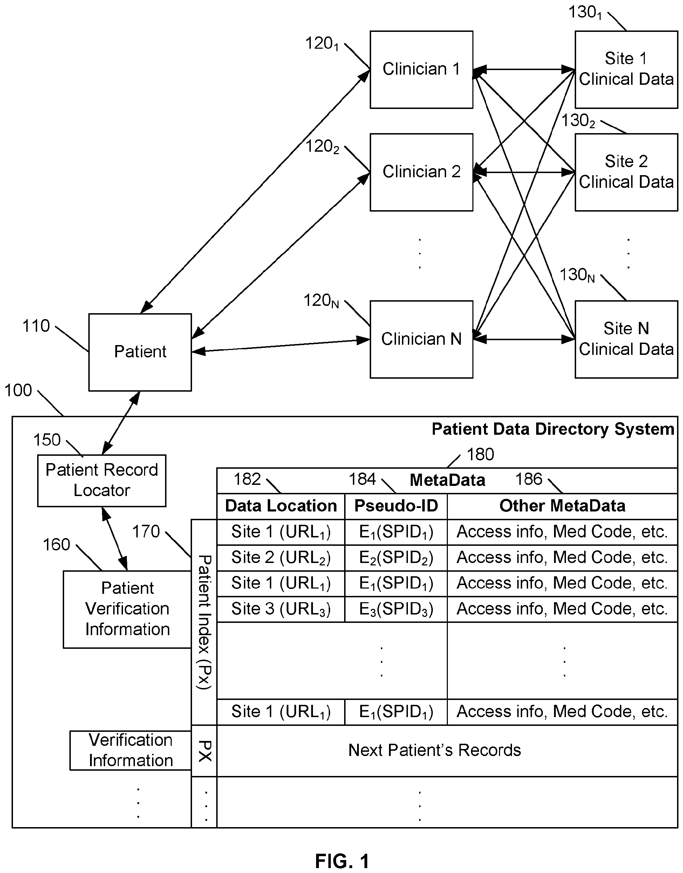

FIG. 1 illustrates an overview of the architecture of an example embodiment of this invention. Over time, a patient 110 may visit multiple clinicians 120₁, 120₂, ... 120_{N}, each of these clinicians being associated with a particular medical site. The clinicians store the patient's clinical data 130₁, 130₂, ... 130_{N} at the corresponding medical site. Although FIG. 1 illustrates a one-to-one correspondence between clinicians and sites for ease of understanding, one of skill in the art will recognize that multiple clinicians may be associated with each site. In like manner, each medical site is illustrated as having a single set of clinical data associated with the patient, whereas one of skill in the art would recognize that a medical site may have a variety of different departments, each having clinical records associated with the patient. As used herein, the term 'site' includes 'sub-sites', such as the different departments at a site. Also for ease of understanding, the terms 'clinician' and 'site' may be used interchangeably when referring to actions taken at each site.

To preserve the patient's privacy, each site (or sub-site) identifies the patient using a pseudo-ID, such as a patient number or other identifier associated with the patient's clinical records, and access to the patient's clinical data requires knowledge of that patient's pseudo-ID. Typically, a patient will identify himself/herself to the clinician, and on the first visit to the medical site, the medical site will assign a pseudo-ID for this site to the patient. Thereafter, the clinician will identify the patient's clinical data using the site-assigned pseudo-ID corresponding to the patient. Note that each site (or sub-site) may assign a different pseudo-ID to the patient.

When the clinician enters the patient's clinical data as a record in the site's storage facility, the clinician, or the site, creates 'metadata' that is associated with the record. In particular, the metadata includes an identification of the record that facilitates access to the record, such as the URL of the particular site, and, optionally, an indication of the location of the record at this URL. Additionally, because the patient's pseudo-ID at the site is required to access the patient's clinical data at the site, the metadata associated with the record also includes the patient's pseudo-ID at this particular site. Preferably, the site-specific pseudo-ID of the patient is encrypted by the site, and this encrypted pseudo-ID is included in the metadata. In this manner, the site-specific pseudo-ID of the patient is known only to the particular site.

Additional metadata may include information that characterizes the clinical data that is stored at the record, such as an identification of the anatomic region, a classification of the illness being treated, the date of treatment, and so on. In some embodiments, a set of pre-defined medical 'codes' may be used to classify the content of the record. This additional metadata may be used to 'filter' access to the record. For example, the user may grant a clinician access to only those records related to a particular anatomic region, or records related to a particular range of dates, and so on.

This metadata for each record of the patient's clinical data at each site is stored in a Patient Data Directory System (PDDS) 100. The PDDS 100 may be used to store the metadata of multiple patients, and the PDDS 100 includes a patient record locator 150 that identifies the particular patient based on a set of patient verification information 160, discussed further below. As illustrated in FIG. 1, the metadata 180 of each record includes a data location 182, such as the site's URL and, optionally, an indication of where, at the site, the record is located. The metadata also includes the site's pseudo-ID (SPID) 184 assigned to the patient, preferably encrypted by the site, and other metadata 186. The other metadata 185, as discussed above, includes any further information required to access the record, as well as information that characterizes the content of the record, such as a medical code.

One of skill in the art will recognize that the particular data structure of the metadata 180 at the PDDS 100 may differ from the linear structure illustrated in FIG. 1. For example, the data structure may be hierarchical, with the site information (site location 182 and SPID 184) at a higher level, and each of the specific 'other' metadata 186 associated with each new set of clinical data associated with the patient at this site being 'sub-records' below the site location information. Other structures, including linked-lists and others, may also be used.

Each time a new set of clinical data 130 for the patient 110 is stored at a site, the metadata 180 related to this clinical data is stored at the PDDS 100, indexed by an identification (Px) 170 of the particular patient. In the example of FIG. 1, the metadata 180 for the patient 110 indicates that the patient initially visited Site₁, then visited Site₂, then revisited Site₁, then visited Site₃, etc.

In embodiments of this invention, access to the metadata stored at the PDDS is controlled by each patient. At the patient's first visit to a first clinician, the first clinician 'registers' the patient at the PDDS 100, and in conjunction with the patient 110, provides the patient verification information 160 to facilitate secure access to the metadata 180 that is subsequently added to the patient's record at the PDDS 100.

Because the patient verification information 160 is designed to use secure techniques for granting access to only verified patients, the patient is able to maintain control of the storage and retrieval of the patient's metadata. The patient verification information 160 may include one or more keys used to represent the patient in the PDDS and to link each of the patient's plurality of records. A privacy-preserving cryptographic protocol based on the one or more multiple keys is used by the PDDS to limit access to the identified patient. The one or multiple keys may comprise one or multiple random keys generated by the patient and stored in PDDS. Preferably, the one or more multiple keys do not reveal identification information of the patient.

In some embodiments, the privacy-preserving cryptographic protocol may require a one-time setup and subsequent challenge-response authentications. The one-time setup may require the patient to generate a public key and a private key of a public/private key pair. The private key is stored by the patient and the public key is stored in the PDDS, preferably by the clinician when the patient is first registered at the PDDS.

As illustrated in FIG. 1, each clinician 120 may access records at multiple sites 130, provided that the patient provides the metadata of each accessible site to the clinician. Typically, each clinician has read and write access to the site that the clinician is associated with, as indicated by the double-head arrows between each clinician and the associated site. Each clinician typically has read-only access to the sites that the clinician is not associated with, as indicated by the single-head arrow between each clinician and each non-associated site. As detailed further below, each clinician that is authorized to access information at the clinical sites is provided an authorization token, and provides the authorization token to each specific clinical site to gain access and retrieve the clinical data of the patient at each specific clinical site.

When a clinician desires access to the patient's records at multiple sites, the clinician submits a request to the patient, and the patient accesses the PDDS to retrieve the metadata related to the request. In some cases, the patient may grant access to the entirety of the records associated with the patient, and will correspondingly provide all of the patient's metadata to the clinician. The 'request' by the clinician may be implicit, wherein, for example, before a scheduled visit with the clinician, the patient may anticipate that the clinician will request the patient's metadata, and accesses the PDDS to retrieve the metadata before the visit.

In some cases, as noted above, the patient will grant access to only a select set of the patient's records. In such cases, the patient will only provide the metadata related to a selection criteria, such as a span of dates, an anatomical region, etc. The selection criteria may also include exception criteria, wherein the user identifies a class of records that should not be provided to the clinician. Not illustrated in FIG. 1, the PDDS, or the patient's device, will include a filtering system wherein the patient provides the selection criteria to the filtering system, and the filtering system provides only the metadata that satisfies the selection criteria to the patient, for subsequent communication to the requesting clinician.

In some embodiments, the PDDS may be configured to store pre-defined selection criteria for some or all of the clinicians that have access to the patient's records and will filter any further data access requests based on these pre-defined selection criteria, unless specifically directed otherwise by the patient.

Upon receipt of the requested metadata from the patient, the clinician accesses the patient's records at each site identified in the received metadata. The clinician provides the (preferably encrypted) site-specific pseudo-ID (SPID) for the patient, as well as any further access information in each metadata entry to the identified site in the metadata. Given the access information, each site will decrypt the pseudo-ID and provide the clinical records of the identified patient to the clinician, but only if the access information is proper and the records at the specified location are associated with the pseudo-ID of the patient. One of skill in the art will recognize that if the metadata indicates that the records are stored at the site associated with the clinician, a different protocol may be used to access the locally stored clinical records.

Typically, the clinician will review the received clinical records, examine the patient as deemed necessary, then create a new clinical record that is stored at the clinician's associated site. Metadata related to this new clinical record is created and the clinician provides this metadata to the patient with a record-update request. If the patient approves this request, the patient provides this metadata to the PDDS for storage as the next entry in the patient's metadata records. To further protect access to the patient's clinical data, the patient may encrypt some or all of the metadata, particularly the site location and the pseudo-ID in the metadata that enables access to the data at the medical site. In some embodiments, as detailed further below, the PDDS may be configured to receive or retrieve the metadata directly from/to the clinician, upon approval by the patient.

As noted above, embodiments of this invention include security measures for protecting the security of the patient's records. A fundamental security criteria assures that only qualified/certified clinicians have access to the patient's clinical records stored at the multiple sites.

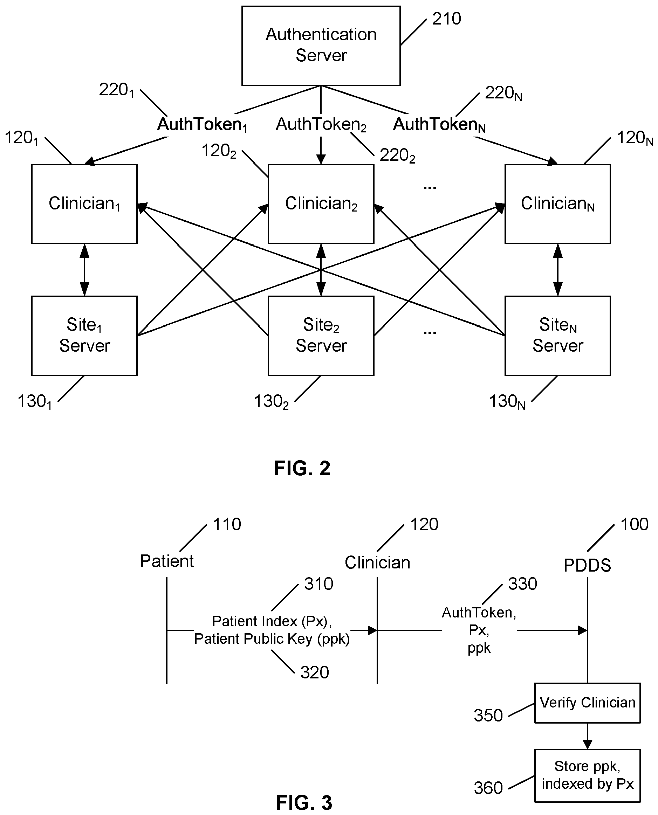

FIG. 2 illustrates an example clinician authentication system. In this embodiment, each clinician 120 authenticates himself/herself to an Authentication Service Provider. The actual implementation of this embodiment could be in multiple ways, including for example, an authentication server installed on a private cloud or one of the healthcare sites, which serves only for clinicians from a set of federated sites. In other embodiments, the Authentication Service Provider could be a well-known IdP (Identity Provider), such as Google, Microsoft, etc., which provides the functionality of federated authentication. Similarly the Authentication Service Provider could be a DID/SSI (Decentralized Identity/Self Sovereign Identity) that relies on some distributed storage or ledger to provide such an identity and authentication service.

In the example of FIG. 2, during the authentication process, a clinician 120; sends an authentication request to the Authentication Server 210 and, if the authentication is successful, receives a corresponding authentication token AuthTokenᵢ. The AuthToken will typically contain an identification of the clinician, signed by the Authentication Service Provider, and a cryptographic key, such as a public key of a public-private key pair assigned to the clinician. The AuthToken may also contain ancillary information, such as the site associated with the clinician, the clinician's specialty, etc. Later, when the clinician needs to access the PDDS or other sites, the clinician sends the AuthToken to the site, and proves his/her identity using a conventional cryptographic exchange based on the information contained in the AuthToken.

Preferably, access to the patient's clinical data is controlled by the clinical sites. In such embodiments, the Authentication Server authenticates the identity of the clinician, and the clinical sites have a list of clinicians authorized to access their records (or authorized to access a subset of their records). With the AuthToken, the clinician requests access to the patient's data at the clinical site, and the clinical site verifies the identity of the clinician based on the AuthToken. Upon successful verification, the clinical site determines whether this authenticated clinician is on their list of authorized clinicians for the requested records. The "list" of authorized clinicians may be an itemized list of each authorized clinician, or it may be in a more generalized form, such as "all authenticated clinicians at sites A, B, or C", or "all authenticated clinicians having specialty X". In some embodiments, the AuthToken may also serve as both an authentication of identity and an access authorization, wherein the list of authorized clinicians is in the form of "all authenticated clinicians".

In embodiments of this invention, "Open Authorization 2.0" (OAuth2) may be used to provide the AuthToken to the clinician. In OAuth2, the token is termed "Access Token". The clinician requests the Access Token from an "Authorization Server", and submits his/her credentials. The Authorization Server submits the clinician's credentials to a "Resource Owner"; in this case, the Resource Owner is an organization to which each of the medical sites ("Resource Servers") and the PDDS belong. The Resource Owner validates the clinician's credentials, and notifies the Authorization Server whether the clinician is authorized to obtain data stored at the Resource Servers. If authorized, the Authorization Server sends the Access Token to the clinician, which serves as the AuthToken of this invention.

One of skill in the art will recognize that the term "authentication token" is used herein in the generic sense, comprising any form of identification information that enables a clinician to prove his/her identity.

FIG. 3 illustrates an example message exchange for initiating a patient's access to the PDDS. This process is performed when the patient first visits a participating clinician. Any of a variety of access techniques may be used by the PDDS, such as having the patient choose a "user ID" and "password", or other conventional cryptographic verification techniques.

In the example embodiment represented in FIG. 3, the patient is in possession of a public-private key pair: a "ppk" - the patient's public key, and a "psk" - the patient's secret (private) key. These keys will be used to authenticate the patient each time the patient accesses the PDDS, typically via a "challenge"-"response" exchange between the PDDS and the patient, detailed further below. To facilitate access, the patient may choose a random number, such as a PIN, that the PDDS will use as an index (Patient Index, Px) to the multiple patients' records at the PDDS.

The patient 110 provides the Patient Index, Px 310, and the Patient's Public Key (ppk) 320 to the Clinician. The clinician communicates 330 the clinician's AuthToken, the Px, and the ppk to the PDDS 100. The PDDS 100 verifies 350 the clinician based on the AuthToken, and if the clinician is verified, the PDDS 100 creates an account for the patient, and stores 360 the ppk for this account, indexed by Px. Optionally, the patient may include an expiration date for the patient's public key, and this expiration date is communicated to the PDDS 100.

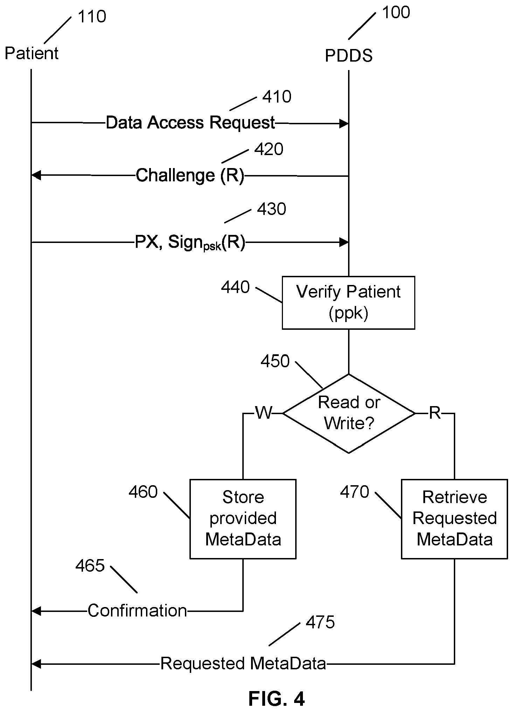

FIG. 4 illustrates an example flow diagram for providing access to the PDDS by the patient. Any number of techniques may be used to grant access to the patient, such as a log-in and password protocol, and others. In the example embodiment of FIG. 4, a conventional challenge-response protocol is used.

At 410, the patient provides a Data Access Request 410 to the PDDS 100. Typically, the Data Access Request 410 will be provided by the clinician to the patient, and will specify either the metadata that is to be written to the PDDS 100 (i.e. the metadata for a newly created clinical record at the clinician's site), or a request to read a set of metadata. As discussed above, the request may be filtered by the patient to provide only selected metadata to the clinician, such as metadata related to a particular anatomical region, a particular diagnosis, a particular time-frame, etc. This filtering may be performed at the patient's device, or the data access request 410 may include filtering criteria for the PDDS 100 to apply. Or, as noted above, the PDDS 100 may be configured with pre-defined filtering criteria for the clinician.

In response to the data access request 410, the PDDS 100 issues a challenge 420, typically in the form of a random number R. In response to the challenge, the patient signs the challenge using the patient's secret key (psk), and communicates 430 this signed challenge Signₚₛₖ(R), and the PDDS finds and verifies the patient based on the PDDS's storage of patients' public keys (ppk). The PDDS 100 could search through all of its patient accounts to find a patient public key (ppk) that corresponds to the patient having the patient secret key (psk). However, in this example embodiment, the patient communicates his/her patient index (Px), and the PDDS 100 only searches through the patient accounts having that patient index to find the patient having the patient public key that corresponds to the patient secret key that was used to sign the challenge (Signₚₛₖ(R)).

Upon finding/verifying the patient based on the signed challenge, the PDDS 100 determines whether the data access request 410 is for a reading of the stored metadata, wherein the clinician is a 'data requestor', or for a writing of a new set of metadata, wherein the clinician is a 'data provider' at 450. If the request is to write a new set of metadata, the PDDS 100 adds this new set of metadata provided by the clinician to the patient's account. As noted above, the patient may encrypt some or all of the metadata before submitting it to the PDDS 100 for storage.

If the data access request 410 is for a reading of the metadata, the PDDS 100 retrieves the metadata requested by the clinician, at 470, and communicates 475 the requested metadata to the patient. If the metadata was encrypted by the patient, the patient decrypts the metadata before sending it to the requesting clinician. As noted above, the requested metadata may be select metadata based on filtering using the patient's selection criteria. Alternatively, the PDDS 100 could be configured to always return all of the patient's metadata to the patient, and the patient may subsequently apply the selection filtering before communicating the selected metadata to the clinician.

One of skill in the art will recognize that the PDDS 100 could be configured to interact with both the clinician and the patient, to avoid having the patient relay the metadata to and from the clinician. In this case, the clinician could initiate the data access request 410 while simultaneously notifying the patient of this request. If the patient concurs, the patient will respond with the challenge response of Px, Signₚₛₖ(R) 430; otherwise, in the absence of a proper challenge response, the PDDS 100 will notify the clinician that the request 410 has not been approved by the patient. In addition to the challenge response 430, the patient 110 may also provide selection filtering criteria to the PDDS 100 for each data access request 410.

The PDDS 100 may also be configured to send the Confirmation 465 and/or the requested metadata 475 directly to the clinician, unless directed otherwise by the patient.

As noted above, the metadata provided to the PDDS 100 may be encrypted by the patient, preferably using a different key than the patient's key(s) that is used to access the PDDS 100.

In some embodiments, some or all of the metadata, and particularly the site location (SURL) and the site's pseudo-ID for the patient (SPID), may be encrypted using a conventional symmetric key to reduce computation overhead for encryption and decryption.

In other embodiments, particularly if the PDDS is configured to transmit the metadata directly to the clinician, a conventional Proxy Re-Encryption (PRE) technique may be used. Proxy re-encryption allows a first message encrypted by a first user using a first public key, to be transformed by a proxy device into a second message that a second user can decrypt, without the proxy device having access to the plain-text (unencrypted) message.

In this case, the patient acquires a public-private key pair (skₚ, pkₚ) and the clinician acquires a public-private key pair (sk_{c}, pk_{c}). During the writing process, the patient encrypts the metadata using the patient's public key (pkₚ) and stores the encrypted metadata E_{PKp}(M) on the PDDS.

When the clinician requests the metadata, the clinician sends his/her public key pk_{c} to the patient. If the patient approves the request, the patient uses a proxy re-encryption scheme to create a re-encryption key based on the patient's private key skₚ and the clinician's public key pk_{c}. The patient sends this re-encryption key to the PDDS with the data access request. The PDDS retrieves the requested patient-encrypted metadata and uses the re-encryption key to transform the patient-encrypted metadata into re-encrypted metadata that can be decrypted by the clinician's private key sk_{c}. The PDDS sends this re-encrypted metadata to the clinician, who then decrypts the metadata using the clinician's private key sk_{c}.

Preferably, the proxy re-encryption scheme used to provide the re-encryption key is configured to generate a unique re-encryption key for each requested data access, using for example a random number selected by the patient for each request. In this case, the proxy re-encryption scheme may require the patient to communicate ancillary data with the re-encryption key to the PDDS.

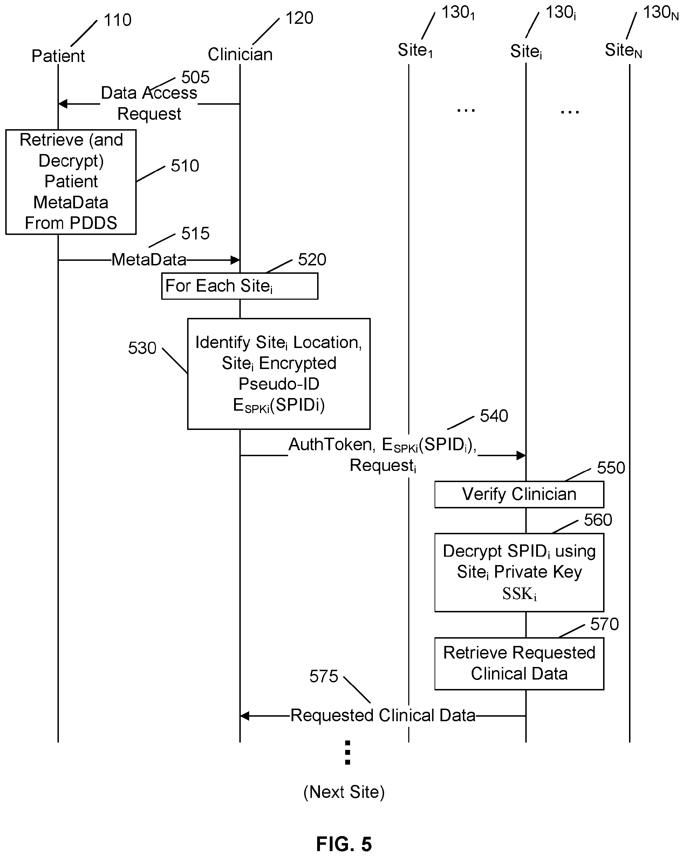

FIG. 5 illustrates an example flow diagram for providing the patient's clinical data to the clinician, based on the patient's metadata from the PDDS.

The clinician 120 initiates the process by sending a data access request to the patient 110. The patient 110 retrieves the requested metadata from the PDDS using the example process of FIG. 4, and sends the metadata to the clinician 120. If the metadata is encrypted, the patient 110 decrypts it and sends the decrypted metadata to the clinician 120. As indicated above, the PDDS may be configured to communicate the metadata to the clinician 120 directly, after the patient 110 approves the requested access by the clinician. Typically, the patient's metadata will include multiple entries, each entry corresponding to clinical data stored at a particular site. The clinician iteratively processes 520 the metadata for each site.

FIG. 5 illustrates the processing of the metadata for a site;; the metadata for each site is processed similarly. However, at the site that is associated with the requesting clinician, a different protocol may be used to access the locally stored clinical data.

At 530, the clinician 120 processes the metadata to identify the Site Location; (Site; URL, or SURLᵢ) and the Pseudo-IDᵢ (SPIDᵢ) for the patient 110 at Site, 130ᵢ. Preferably, the Pseudo-IDᵢ is encrypted by the site; using a public key (SPKᵢ) of the site; when the metadata is first created by the site; and subsequently stored at the PDDS by the patient. The metadata may also include an identification of the clinical data to be retrieved, such as a record pointer or other identifying information.

The clinician 120 sends 540 his/her AuthToken, the encrypted SPIDᵢ, (E_{SPKi}(SPIDᵢ)), and the data request to the identified Site;. Some sites may have multiple public keys; if so, the metadata will include an identification (e.g. index, or Key ID) of which public key was used when the site encrypted the SPID. The data request includes the metadata information that facilitates retrieval of the patient's clinical data, as well as other information.

At 550, the Site; 130ᵢ verifies the clinician 120 based on the AuthToken, and upon successful verification, decrypts 560 the SPIDᵢ using the Site's private/secret key SSKᵢ. Using the decrypted SPIDᵢ, and other metadata information in the request 540, the Site; 130ᵢ retrieves 570 the clinical data of the patient 110, and sends it to the clinician 120 (575).

In embodiments of this invention, the PDDS enables replacement of the key(s) used to verify the patient, as well as replacement of some or all of the stored metadata. For example, a medical site may wish to re-encrypt the encrypted SPID in the stored metadata. This re-encryption may be based on a schedule of key updates for enhanced security. The re-encryption may also be based on discovery of a possible compromise of prior keys, or other security concerns. In like manner, the patient may wish to re-encrypt the patient-encrypted stored metadata for any of a variety of reasons.

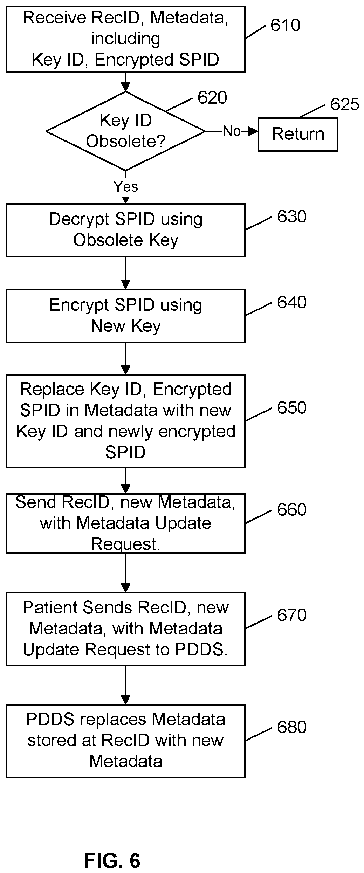

FIG. 6 illustrates an example flow diagram for re-encrypting the site-encrypted pseudo-ID in the stored metadata at the PDDS. The site has multiple keys, each identified by a Key ID (index to keys). In this embodiment, each time a data access request is received at the site, the site determines whether the key is 'obsolete' and should be replaced. This process may be performed before or after the data access request is serviced.

At 610, the site receives the metadata associated with the data request, with an identification of which record (RecID) in the PDDS this metadata is located. This RecID enables the patient to identify at which record the metadata should be replaced by the re-encrypted metadata. Typically, the data request will come from a clinician, but in some embodiments, the site may allow a patient to directly request the clinical records associated with the patient, or at least directly request that the metadata be checked for obsolete keys. Alternatively, the site may initiate this process by requesting receipt of some or all of its metadata stored at the PDDS; this request may be sent to the clinician or the patient.

At 620, the site determines whether the key that was used to encrypt the pseudo-ID is obsolete, based on the Key ID. As noted above, the keys may be determined to be obsolete based on a schedule of replacement keys, or based on the occurrence of particular events. If the key is not obsolete, no action is taken 625.

If the key is obsolete, the site decrypts the SPID using the original, obsolete key at 630, and re-encrypts it using a new key, which is identified by a new Key ID, at 640.

At 650, the site replaces the Key ID and encrypted SPID with the new Key ID and newly encrypted SPID in the metadata. This replacement metadata is communicated to the patient with a Metadata Update Request with an identification of the record RecID at which the metadata is to be replaced, at 660. The site may send the replacement metadata and update request to the clinician, for forwarding to the patient.

At 670, the patient (or site) sends the replacement metadata and update request to the PDDS, and the PDDS replaces the stored metadata at the record identified by RecID with this replacement metadata, at 680.

One of skill in the art will recognize that a similar process may be used to re-encrypt other data. For example, if the stored metadata is encrypted by the patient using a public/private key pair, and the patient wants to re-encrypt the metadata with a new public/private key pair, the patient can send the new public key to the PDDS and request each encrypted metadata record from the PDDS. The patient will decrypt the encrypted metadata using the patient's original private key, re-encrypt it using the patient's new public key, and return the newly encrypted metadata with a metadata update request to the PDDS.

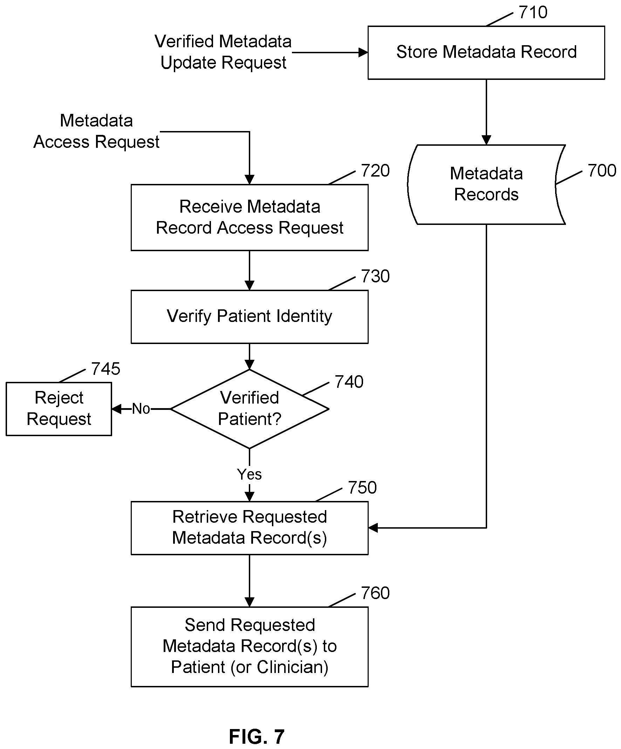

FIG. 7 illustrates a flow diagram of an example method of storing and accessing metadata records of a patient at a PDDS.

At 710, the method comprises storing 710 metadata records 700 of a patient at the PDDS. As detailed above, appropriate verifications are performed to verify that the metadata is provided by the patient.

As also detailed above, the metadata is associated with the clinical records of the patient at a plurality of clinical sites. The metadata is provided by the clinical site for each new clinical record stored at the site, and includes an identification of the clinical site and a site-specific pseudo-ID associated with the patient at the clinical site. The pseudo-ID is necessary to access the clinical data at the clinical site, and is preferably encrypted by the clinical site. The metadata may also include other information that facilitates selective filtering of the records by the patient before sending the metadata to a particular data requestor.

Subsequent to the storing of the metadata records 700, the PDDS may receive 720 a metadata record access request from the patient. This access request may include a request for one or more metadata records. The patient's identity is verified 730, as detailed above, and if verified 740, the requested metadata record(s) is retrieved 750 from the stored metadata records 700.

At 760, the requested metadata records are sent to the patient, for forwarding to the requesting clinician, or, as detailed above, sent directly to the clinician if authorized by the patient. Not illustrated in FIG. 7, the patient may selectively limit/filter the metadata that is communicated to the requesting clinician. This filtering may be performed at the PDDS, or at the patient's device. If performed at the PDDS, the patient may include the filtering criteria in the metadata access request.

In a preferred embodiment, a data access tracking system is provided to record interaction messages among patients, clinicians, sites, and the PDDS as access records. Each access record preferably includes information associated with each interaction message and a timestamp. The interaction messages may include, for example, a registration request, a record access request, a record update request, a clinical data access request, a request approval, and a request denial.

The data access tracking system may be an independent system that receives the interaction messages from the various components of the distributed clinical data management system of this invention, or it also may be distributed among the various components. For example, the Authentication Server 210 may maintain a log that identifies each clinician, the date of issuance of the AuthToken, and other information such as the site associated with the clinician.

The PDDS 100 may maintain a log related to the patient, a log related to the clinician, and a log related to metadata access. The patient log may contain an identification of each patient, a record of changes to each patient's index Px, and other changes. The clinician log may contain information extracted from the AuthToken and information that uniquely identifies each clinician. The metadata access log may contain information related to each access to the patient's metadata, including an identification of the patient and requesting clinician, a date-time stamp, and an identification of the metadata that was read or written. In like manner, each Site 130 may maintain a log that identifies the patient, the clinician, a date-time stamp, and an identification of which clinical data was read or written. In such a distributed embodiment, the data access tracking system includes a management system that collects and consolidates the information (interaction messages) from the various logs.

The data access tracking system includes a user interface that enables review of the access records. The review of the access records may be selectively limited. For example, the patient may have access to all interaction messages related to the patient or the patient's records, whereas the clinician may only have access to interaction messages in which the clinician was a party.

While the invention has been illustrated and described in detail in the drawings and foregoing description, such illustration and description are to be considered illustrative or exemplary and not restrictive; the invention is not limited to the disclosed embodiments.

Of particular note, although the invention is presented as comprising a distinct PDDS 100 that is separate from the patient's device 110 and the clinical sites 130, one of skill in the art will recognize that the PDDS 100 may be a centralized service on a cloud server, or within a server at a select clinical site 130. In like manner, the PDDS can be a privately managed application that is embodied in the patient device 110. That is, for example, each patient may be provided a PDDS "app" for storing his/her individual metadata records. This PDDS app may be embodied as an application on the patient's personal computer, or, for example, as a "digital wallet" on the patient's mobile device. An advantage of providing an individualized PDDS to each patient is that such an integration will facilitate automation of tasks such as receiving, decrypting, filtering, and forwarding the metadata to the clinician. Such an embodiment also reduces the risks associated with a data breach at a centralized PDDS.

Additionally, although the invention is presented in the context of having the patient involved in each metadata access request, it is possible to operate the invention in an embodiment wherein the patient authorizes one or more clinicians to access the PDDS 100 at any time. In this case, the patient's approval is only required for creating this authorization, or for data access requests that have not been pre-approved. In such an embodiment, the clinician may contact the PDDS 100 directly, and if the clinician has been pre-approved, the PDDS 100 provides the metadata directly to the clinician.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Also in the claims, the expression "at least one of A, B, and C" means "at least one of A, B, and/or C". A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A patient data directory system (PDDS) comprising:
a plurality of records associated with a patient, and
an access control system that controls access to the plurality of records,
wherein the access to the plurality of records is controlled by the patient,
wherein the patient can approve or deny access to the clinical data of the patient by each of a plurality of data requestors,
wherein the plurality of records comprises metadata that is associated with clinical data of the patient that is stored at a plurality of clinical sites,
wherein each clinical site identifies the patient using a site-specific pseudo-ID (SPID) of the patient,
wherein the metadata of each record identifies a location of the patient's clinical data at a corresponding clinical site, and the SPID of the patient at the corresponding clinical site,
wherein the access control system comprises an identity verification process to verify that a user of the system is the patient that controls access the metadata records, and
wherein if the user is verified as being the patient, and data access is approved for the data requestor, the metadata is communicated to the data requestor to enable the data requestor to access to the clinical data of the patient at the corresponding clinical site based on the SPID of the patient at the corresponding clinical site.

2. The PDDS of claim 1,
wherein one or multiple keys are used to represent the patient in the PDDS and to link each of the patient's plurality of records,
wherein the one or multiple keys comprise one or multiple random keys provided by the patient and stored in PDDS,
wherein the one or more multiple keys do not reveal identification information of the patient, and
wherein the verification mechanism uses a cryptographic protocol based on the one or more multiple keys.

3. The PDDS of claim 2, wherein the PDDS enables the patient, after passing identity verification, to change the one or multiple keys that are used to represent the patient in the PDDS and to link each of the patient's plurality of records.

4. The PDDS of claim 1,
wherein the verification mechanism comprises a cryptographic protocol that requires a one-time setup and subsequent challenge-response authentications,
wherein the one-time setup requires the patient to provide a public key and a private key of a public/private key pair, and
wherein the private key is stored by the patient and the public key is stored in the PDDS via a data provider who registers a new SPID to the PDDS for the patient.

5. The PDDS of claim 1,
wherein each of a plurality of data providers is provided an authentication token, and
wherein the data provider provides the authentication token to the PDDS to register a new SPID for the patient at the PDDS.

6. The PDDS of claim 1,
wherein each of a plurality of data requestors is provided an authentication token, and
wherein the data requestor provides the authentication token to each clinical site to gain access and retrieve the clinical data of the patient at each clinical site.

7. The PDDS of claim 1,
wherein a data provider provides the metadata associated with the clinical data of the patient,
wherein the data provider sends a record-update request including the metadata to the patient,
wherein the patient approves or denies the request, and
wherein if the request is approved, the patient encrypts the metadata to create patient-encrypted metadata that is stored in the PDDS.

8. The PDDS of claim 1,
wherein the metadata is encrypted by the patient to form patient-encrypted metadata that is stored at the PDDS, and
wherein the PDDS comprises a proxy re-encryption system that transforms the patient-encrypted metadata into re-encrypted metadata that can be decrypted by the data requestor.

9. The PDDS of claim 1,
wherein the metadata is encrypted by the patient to form patient-encrypted metadata that is stored at the PDDS, and
wherein the PDDS sends the patent-encrypted metadata to the patient for subsequent communication of the metadata to the data requestor.

10. The PDDS of claim 1,
wherein the SPID in the metadata that is communicated to the data requestor is encrypted using a cryptographic key owned by the corresponding clinical site, and
wherein the data requestor sends the encrypted SPID to the corresponding clinical site, which can then be decrypted as needed by the clinical site.

11. The PDDS of claim 10,
wherein a clinical site possesses multiple cryptographic keys, each identified by a unique key ID,
wherein the metadata includes the key ID and the SPID encrypted using the key referenced by the key ID, and
wherein the key ID and the encrypted SPID are communicated to the data requester to identify the key used for encryption by the clinical site.

12. The PDDS of claim 11, wherein the PDDS enables replacement of the key ID and the SPID that is encrypted using the key referenced by the key ID with a new key ID and the SPID that is encrypted using the new key ID.

13. The PDDS of claim 1, wherein the PDDS includes a plurality of metadata records for a plurality of patients.

14. The PDDS of claim 1,
wherein the patient provides selective access to the metadata to each data requestor, and
wherein the selective access is based on ancillary information in the metadata,
wherein the ancillary information comprises one or more of:
an anatomical region associated with the clinical data,
a time-frame related to the clinical data, and
a diagnosis associated with the clinical data.

15. The PDDS of claim 1,
wherein a public-private key pair is associated with the patient,
wherein the public key of the public-private key pair is communicated to the PDDS, and
wherein the PDDS system enables the patient, after passing identity verification, to change the public key in the PDDS to a new public key corresponding to a new public-private key pair of the patient.

16. A patient device for use by a patient, comprising:
a communication system for communicating with a data requestor and a patient data directory system (PDDS), and
a user interface;
wherein the PDDS is configured to store records related to clinical data of the patient stored at a plurality of clinical sites and patient verification information,
wherein the records comprise metadata associated with the clinical data at each of the plurality of clinical sites,
wherein the metadata includes an identification of a location of the clinical data at each of the plurality of clinical sites, and a site-specific pseudo-ID (SPID) of the patient,
wherein, when the patient device receives a record-access request from the data requestor:
the patient device, via the user interface, presents the request, and receives an approval or denial response from the patient,
if the request is approved:
the patient device uses the cryptographic system to gain access to the metadata records of the patient at the PDDS,
the patient device uses the communication system to communicate the record-access request to the PDDS,
the patient device uses the communication system to receive, in response to the record-access request, the metadata records of the patient from the PDDS, and
the patient device uses the communication system to communicate some or all of the metadata selected by the patient to the data requestor, to facilitate access to the clinical data of the patient stored at the plurality of clinical sites by the data requestor.

17. The patient device of claim 16, comprising a cryptographic system,
wherein, when the patient device receives a record-update request from the data provider:
the patient device, via the user interface, presents the request to the patient, and receives an approval or denial response from the patient,
the patient device uses the cryptographic system to gain access to the PDDS, and
the patient device uses the communication system to communicate the metadata to the PDDS.

18. The patient device of claim 16, wherein the patient device includes the PDDS.

19. A data provider device for use by a data provider, comprising:
a communication system for communicating with a patient and a clinical site server associated with the data provider;
wherein a patient data directory system (PDDS) is configured to store records related to clinical data of the patient stored at a plurality of clinical sites,
wherein the records comprise metadata associated with the clinical data at each of the plurality of clinical sites,
wherein the metadata includes an identification of a location of the clinical data at each of the plurality of clinical sites, and a site-specific pseudo-ID (SPID) of the patient,
wherein the data provider device uses the communication system to store the patient's clinical data in a clinical site server and receive corresponding metadata in response, and
wherein the data provider device uses the communication system to communicate a record-update request including the metadata to the patient for subsequent submission to the PDDS.

20. A data requestor device comprising:
a communication system for communicating with a patient and a plurality of clinical sites;
wherein the patient has access to a patient data directory system (PDDS) that is configured to store records related to clinical data of the patient stored at the plurality of clinical sites and patient verification information,
wherein the records comprise metadata associated with the clinical data at each of the plurality of clinical sites,
wherein the metadata includes an identification of a location of the clinical data at each of the plurality of clinical sites, and a site-specific pseudo-ID (SPID) of the patient,
wherein the data requestor device uses the communication system to communicate a data access request to the patient,
wherein the data requestor device uses the communication system to receive the metadata associated with the clinical data from the patient, and
wherein the data requestor device uses the communication system to access the clinical data of the patient stored at the plurality of clinical sites based on the metadata.

21. A data access tracking system comprising:
a communication system for communicating with a plurality of patients, data providers, data requestors, clinical sites, and a patient data directory system (PDDS),
a storage system,
a cryptographic system, and
a user interface;
wherein the PDDS is configured to store records related to clinical data of the patient stored at a plurality of clinical sites and patient verification information,
wherein the data access tracking system provides access to access records related to all or selected interaction messages among the plurality of patients, data providers, data requestors, clinical sites, and the PDDS,
wherein each interaction message comprises at least one of: a registration request, a record access request, a record update request, a clinical data access request, a request approval, and a request denial,
wherein each access record includes information associated with each interaction message and a timestamp,
wherein the user interface facilitates review of the access records.

22. A method of managing access to clinical records of a patient, comprising:
storing a plurality of records that facilitate access to the clinical records of the patient,
wherein the plurality of records comprises metadata that is associated with the clinical records of the patient at a plurality of clinical sites,
wherein the metadata of each record comprises an identification of a clinical site of the plurality of clinical sites and a pseudo-ID associated with the patient at the clinical site,
wherein the pseudo-ID is necessary to access the clinical data at the clinical site,
receiving a record access request from a user,
verifying that the user is the patient,
if the user is verified to be the patient, providing the metadata requested in the record access request to at least one of: the patient, or a data requestor that is authorized by the patient to access the clinical data at the clinical site identified in the metadata.

23. A non-transitory computer-readable medium comprising a program that, when executed by a processing system, causes the processing system to perform the method of claim 22.
